(19) Europäisches Patentamt
European Patent Office
Office européen des brevets

(11) **EP 2 168 942 B1**

(12) **EUROPÄISCHE PATENTSCHRIFT**

(45) Veröffentlichungstag und Bekanntmachung des Hinweises auf die Patenterteilung:
**04.07.2012 Patentblatt 2012/27**

(51) Int Cl.:
*C07C 201/08* (2006.01)    *C07C 205/06* (2006.01)

(21) Anmeldenummer: **09011636.9**

(22) Anmeldetag: **11.09.2009**

(54) **Verfahren zur kontinuierlichen Herstellung von Nitrobenzol**

Method for continuous production of Nitrobenzol

Procédé destiné à la fabrication continue de nitrobenzène

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **24.09.2008 DE 102008048713**

(43) Veröffentlichungstag der Anmeldung:
**31.03.2010 Patentblatt 2010/13**

(73) Patentinhaber: **Bayer MaterialScience AG**
**51368 Leverkusen (DE)**

(72) Erfinder:
• **Knauf, Thomas, Dr.**
  **41542 Dormagen (DE)**
• **Racoes, Alexandre**
  **47805 Krefeld (DE)**
• **Dohmen, Wolfgang**
  **47229 Duisburg (DE)**
• **Rausch, Andreas, Dr.**
  **41564 Kaarst (DE)**

(56) Entgegenhaltungen:
EP-A- 0 373 966    EP-A- 0 436 443
WO-A-2009/002734    DE-A1- 19 539 205
US-B1- 6 242 657

**Beschreibung**

Die Erfindung betrifft ein Verfahren zur kontinuierlichen Herstellung von Nitrobenzol durch Nitrierung von Benzol mit Mischsäure enthaltend mindestens 3,0 Gew.% Salpetersäure und mindestens 67,0 Gew.% Schwefelsäure in einem Reaktionsraum, bei dem die Starttemperatur der Reaktion über 100,0°C aber unter 102,0°C liegt, und bei dem Benzol und die Mischsäure mehrmals ineinander dispergiert werden.

Die vorliegende Erfindung betrifft ein kontinuierliches Verfahren zur Herstellung von Nitrobenzol, das im Wesentlichen dem Konzept der adiabaten Nitrierung von Benzol durch ein Gemisch von Schwefel- und Salpetersäure (Mischsäure) entspricht. Ein solches Verfahren wurde erstmals in US-A-225 69 99 beansprucht und ist in heutigen Ausführungsformen beispielsweise in EP 0 436 443 B1, EP 0 771 783 B1 und US 656 2247 B2 beschrieben. Die Verfahren mit adiabater Reaktionsführung zeichnen sich insbesondere dadurch aus, dass über die Außenfläche des Reaktionsvolumens nicht mit technischen Maßnahmen Heiz- oder Kühlenergieströme fließen.

Den beschriebenen adiabaten Verfahren ist gemein, dass die Ausgangsstoffe Benzol und Salpetersäure in einem großen Überschuss an Schwefelsäure zur Reaktion gebracht werden. Die Schwefelsäure nimmt dann die frei werdende Reaktionswärme und das bei der Reaktion gebildete Wasser auf.

Zur Durchführung der Reaktion werden Salpetersäure und Schwefelsäure im Allgemeinen zur sogenannten Mischsäure oder Nitriersäure vermischt bzw. dispergiert und Benzol in diese Mischsäure oder Nitriersäure hinein dosiert, das mit der Salpetersäure im Wesentlichen zu Wasser und Nitrobenzol reagiert. Benzol wird mindestens in der bezogen auf die Salpetersäuremenge stöchiometrischen Menge eingesetzt, bevorzugt aber im 2 - 10%igen Überschuss im Vergleich zur stöchiometrisch benötigten Benzolmenge.

Der Reaktionsraum, in dem Benzol und Salpetersäure zur Reaktion gebracht werden, kann aus einer Anordnung von Rührkesseln, einem Schlaufenreaktor oder aus einem oder mehreren in Reihe oder parallel geschalteten Rohrreaktoren bestehen. Die Rohrreaktoren können zylindrisch oder konisch aufgebaut sein. Es ist vorteilhaft, wenn die Rohrreaktoren rückvermischungsfrei betrieben werden, daher wird die Fließgeschwindigkeit innerhalb des Rohrreaktors so hoch gewählt, dass ein Kolbenströmungsverhalten über den ganzen Reaktor erhalten wird.

Der Reaktionsraum wird im Allgemeinen so bemessen, dass nach dessen Durchströmen ein im Wesentlichen salpetersäurefreies Reaktionsgemisch erhalten wird. Zur Beschreibung des Reaktionsverlaufes in dem Reaktionsraum wird dieser zweckmäßigerweise begrifflich unterteilt in eine **Startzone**, in der die Emulsion aus Benzol und Mischsäure durch Dispergierung erstmals hergestellt wird und in der die Reaktion startet und ggf. mehrfach redispergiert wird und in eine daran anschließende **Endzone**, in der die Reaktion bis zu einem nahezu vollständigen Salpetersäureumsatz abläuft. Dabei beginnt die Startzone räumlich mit der ersten Dispergierung von Benzol und Mischsäure. Auch eine begriffliche Unterteilung in mehrere Zonen ist beliebig möglich. Eine ausführliche Beschreibung der physikalischen und chemischen Vorgänge in einem Nitrierapparat liefert EP 0 708 076 B1.

Im Anschluss an die Endzone des Nitrierreaktors wird das Reaktionsgemisch im Allgemeinen einem Phasentrennapparat zugeführt, in dem sich zwei Phasen bilden, wobei die erste als Rohnitrobenzol bezeichnet wird und die zweite als Absäure, die im Wesentlichen aus Wasser und Schwefelsäure besteht. Gleichzeitig entweichen in dem Phasentrennapparat Gase aus der flüssigen Phase, im Wesentlichen Stickoxide, sowie Wasser- und Benzoldampf. Diese Gase werden im Allgemeinen einem Abgassystem zugeführt.

Die im Phasentrennapparat erhaltene Absäure wird üblicherweise einem Entspannungsverdampfer (auch Blitzverdampfer) zugeführt, in dem bei der Entspannung der Absäure ins Vakuum Wasser verdampft wird und so die Absäure gekühlt und aufkonzentriert wird. Bei adiabater Fahrweise der Nitrierung von Benzol mit Mischsäure ergibt sich der Vorteil, dass die Reaktionswärme der exothermen Reaktion dazu genutzt wird, die Absäure so stark zu erhitzen, dass im Entspannungsverdampfer gleichzeitig wieder die Konzentration und die Temperatur eingestellt werden kann, die die Schwefelsäure vor der Zumischung von Salpetersäure und Benzol aufwies.

Das im Phasentrennapparat erhaltene Nitrobenzol (sog. Rohnitrobenzol) weist als Verunreinigungen noch Schwefelsäure, Wasser, Benzol sowie Nitrophenole und Dinitrobenzol auf, die durch geeignete Aufarbeitungsverfahren wie z.B. Wasch- und Destillationsstufen abgetrennt werden. Eine mögliche Ausführungsform dieser Aufarbeitung ist in EP 1 816 117 A1 beschrieben.

Merkmale der adiabaten Nitrierung von aromatischen Kohlenwasserstoffen sind zum einen, dass die Temperatur der Reaktionsmischung proportional zum Reaktionsfortschritt, also proportional zum Salpetersäureumsatz ansteigt. Dadurch wird zum zweiten eine Temperaturdifferenz zwischen der Temperatur der vermischten Edukte vor Einsetzen der Reaktion (folgend als Starttemperatur bezeichnet) und der Temperatur der Reaktionsmischung nach mindestens 99% Salpetersäureumsatz (folgend als **Reaktionsendtemperatur** bezeichnet) erhalten. Dem Fachmann ist bekannt, dass die hier als Starttemperatur bezeichnete Größe im Allgemeinen vorteilhaft unmittelbar nach der Eindosierung des Benzols in die Mischsäure, d.h. vor der ersten Dispergierung, gemessen werden kann und die hier als Reaktionsendtemperatur bezeichnete Größe in dem Zulauf des Phasentrennapparates. Die Differenz zwischen Start- und Reaktionsendtemperatur hängt von der Art des nitrierten Kohlenwasserstoffes ab und von dem volumetrischen Verhältnis in dem der aromatische Kohlenwasserstoff und die Mischsäure eingesetzt wurden. Ein hohes volumetrisches Verhältnis von aromatischem Kohlenwasserstoff und Mischsäure (auch **Phasenverhältnis** genannt) ergibt eine hohe Temperaturdifferenz und hat den Vorteil, dass eine hohe Menge des aromatischen Kohlenwasserstoffs pro Zeiteinheit umgesetzt wird.

In der industriellen Praxis ist die Reaktionsendtemperatur im Falle der Nitrierung von Benzol durch sicherheitstechnische Kriterien auf ca. 135 - 145°C limitiert, wobei Faktoren, wie thermische Zersetzung der Produkte und Dampfdruck der Produktmischung und damit die Abgastemperatur und -menge die ausschlaggebende Rolle spielen. Angesichts dieser vorgegebenen Obergrenze erschien es bislang nahe liegend die Starttemperatur möglichst niedrig zu wählen, da dadurch eine höhere Temperaturdifferenz bis zur großtechnisch anwendbaren Reaktionsendtemperatur vorliegt und deshalb hohe Raum-Zeit-Ausbeuten realisierbar erschienen.

Wichtigstes Kriterium zur Beschreibung der Güte eines adiabaten Verfahrens zur Nitrierung aromatischer Kohlenwasserstoffe ist der Gehalt des Produktes an unerwünschten Nebenprodukten der Reaktion, die durch mehrfache Nitrierung oder Oxidation des aromatischen Kohlenwasserstoffes oder des Nitroaromaten gebildet werden. Im Falle der Nitrierung von Benzol wird dabei stets der Gehalt an Dinitrobenzol und an Nitrophenolen, insbesondere des als besonders brisant einzustufenden Trinitrophenols (Pikrinsäure) diskutiert.

Um Nitrobenzol mit besonders hohen Selektivitäten zu erhalten, wurde die Art der zu verwendenden Mischsäure detailliert festgelegt (EP 0 373 966 B1, EP 0 436 443 B1 und EP 0 771 783 B1), sowie zahlreiche Vorschläge gemacht, wie die erstmalige Mischung und die wiederholte Mischung (Redispergierung) von Benzol in die Mischsäure erfolgen kann (EP 0 373 966 B1, EP 0 489 211 B1, EP 0 771 783 B1, EP 0 779 270 B1, EP 1 291 078 A2 und US 6 562 247 B2). Auch wurde darauf hingewiesen, dass der Gehalt an Nebenprodukten durch die Höhe der Endtemperatur bestimmt wird (siehe EP 0 436 443 B1, Spalte 15, Z. 22-25) und dass ein hoher Anfangsumsatz vorteilhaft ist für eine hohe Selektivität und dass diese dann erreicht wird, wenn zu Beginn der Reaktion eine optimale Durchmischung herbeigeführt wird (EP 0 771 783 B1, Absatz 0014).

Die geforderte Durchmischung wird in EP 0 771 783 B1 durch einen rotierenden Treibstrahl erreicht. Der Einsatz von Dispergierelementen wie beispielsweise in EP 0 489 211 B1 und EP 0 436 443 B 1 beschrieben, wird in EP 0 771 783 B1 als nicht geeignet angesehen (Absätze 0012 bis 0015).

Allen zitierten Verfahren gelingt es Nitrobenzol durch Nitrierung von Benzol mit Mischsäure zu erhalten, wobei eine adiabate Fahrweise angewandt wird, um die Schwefelsäure mit der Reaktionswärme zu erhitzen und so die Reaktionswärme zur Aufkonzentrierung der Absäure verwenden zu können. Allen beschriebenen Verfahren gelingt es ein Rohnitrobenzol herzustellen, das einen geringen Gehalt an Nebenprodukten aufweist, also nur zwischen 100 - 300 ppm an Dinitrobenzol und 1500 - 2500 ppm an Nitrophenolen enthält, wobei Pikrinsäure einen Anteil von 10 - 50% an den Nitrophenolen annehmen kann.

Das Patent US-B1-6 242 657 befasst sich mit der Herstellung aromatischer Nitroverbindungen durch Reaktion aromatischer Verbindungen mit Nitriersäuren in Gegenwart von oberflächenaktiven Substanzen in Gehalten zwischen 0.5 und 20000ppm.

Die Offenlegungsschrift DE 195 39 205 A1 befasst sich mit einem Verfahren zur selektiven Einführung einer Nitrogruppe in einkernige Aromaten mit Gemischen aus Salpetersäure und Schwefelsäure (Mischsäure, Nitriersäure), bei dem der zu nitrierende Aromat einem zentralen Treibstrahl der Mischsäure so zugeführt wird, dass er diesen umhüllt.

Das erfindungsgemäße Verfahren zur kontinuierlichen Herstellung von Nitrobenzol durch Nitrierung von Benzol mit Mischsäure enthaltend mindestens 3.0 Gew.% Salpetersäure und mindestens 67,0 Gew.% Schwefelsäure in einem Reaktionsraum, bei dem die Starttemperatur der Reaktion über 100,0°C aber unter 102.0°C liegt, und bei dem Benzol und die Mischsäure mehrmals ineinander dispergiert werden, ist nicht Gegenstand dieser Schriften.

Die Reinheit des Rohnitrobenzols ist unbenommen weiterhin von entscheidender Bedeutung für die industrielle Produktion. Jedoch ergibt sich vor dem Hintergrund des steigenden Bedarfes an Nitroaromaten, insbesondere zur Herstellung von aromatischen Aminen und aromatischen Isocyanaten, ein weiteres Ziel: Dies ist die Bereitstellung der Möglichkeit hohe Mengen dieser Verbindungen in möglichst kompakten Reaktionseinrichtungen herzustellen.

Der umfangreiche Stand der Technik geht kaum auf die Dimensionierung der vorgeschlagenen Mischeinrichtungen und Reaktoren ein. Lediglich EP 0 779 270 B1 nennt hierzu konkrete Zahlen eines Laborreaktors.

Eine Kenngröße zur Beschreibung des Verhältnisses zwischen produzierbarer Menge und Größe der Reaktionseinrichtung ist die Raum-Zeit-Ausbeute. Sie berechnet sich als Quotient aus der produzierbaren Menge der Zielverbindung pro Zeiteinheit und dem Volumen der Reaktionseinrichtung. Im vorliegenden Falle der Nitrierung von Benzol berechnet sich die Raum-Zeit-Ausbeute zweckmäßiger Weise als Quotient aus der Produktion von Nitrobenzol in metrischen Tonnen pro Stunde und dem Volumen des Reaktionsraumes, wobei der Reaktionsraum als der Raum definiert wird, der mit der ersten Dispergierung von Benzol und Mischsäure beginnt und innerhalb dessen die Reaktion bis zu einem Grad von mindestens 99% abgeschlossen ist. Die Verweilzeit der Reaktionsmischung, bestehend aus aromatischer Verbindung und Mischsäure, innerhalb des Reaktionsraumes ist die **Reaktionszeit**.

$$\text{Raum-Zeit-Ausbeute } [t / m^3 \, h] = \text{produzierte Menge } [t / h] \, / \text{ Reaktionsraum } [m^3]$$

**EP 2 168 942 B1**

Eine hohe Raum-Zeit-Ausbeute ist für die industrielle Anwendung eines Verfahrens von Vorteil, da dadurch kompakte Reaktionseinrichtungen gebaut werden können, die sich durch ein geringes Investitionsvolumen pro Kapazität auszeichnen.

Ziel der vorliegenden Erfindung ist es also, ein Verfahren bereit zu stellen, mit dem zum einen eine hohe Raum-Zeit-Ausbeute erzielt werden kann und mit dem zum anderen die geforderte Produktqualität gewährleistet bleibt.

Überraschend wurde gefunden, dass die Raum-Zeit-Ausbeute erst dann unter Beibehaltung der Produktqualität signifikant gesteigert werden kann, wenn alle reaktionsbeschleunigenden Faktoren - dies sind die Zusammensetzung der Mischsäure, die Starttemperatur und die Güte der Durchmischung - so aufeinander abgestimmt werden, dass zu Beginn der Reaktion in einer Startzone, die höchstens 13 Vol.% des Reaktionsraumes einnimmt mindestens 60% der eingesetzten Salpetersäure mit Benzol zu Nitrobenzol abreagiert ist.

Zur Steigerung der Raum-Zeit-Ausbeute sieht der Stand der Technik vor, das Phasenverhältnis zu erhöhen und die Starttemperatur abzusenken, um eine höhere Temperaturdifferenz bis zur großtechnisch anwendbaren Reaktionsendtemperatur zu erhalten. Es wurde überraschend gefunden, dass es mit diesen Maßnahmen nicht gelingt mit der Steigerung der Raum-Zeit-Ausbeute für Nitrobenzol auch die Produktqualität und die erforderliche Prozesskontrolle beizubehalten.

Um bei gleich bleibender Produktqualität Raum-Zeit-Ausbeuten von mehr als 5 t / m³ h zu realisieren, ist es erforderlich im Vergleich zu einem Verfahren gemäß dem Stand der Technik zwei wesentliche Maßnahmen zu ergreifen:

1. Erhöhung der Starttemperatur. Damit wird zwangsläufig eine höhere Reaktionsendtemperatur in Kauf genommen, was aber bei Anwendung der Kombination der hier geschilderten Maßnahmen nur in zu vernachlässigendem Maße zu dem in EP 0 436 443 B 1 geschilderten Effekt führt, dass der Gehalt an Nebenprodukten ansteigt.

2. Die Reaktionsmischung muss unmittelbar nach der Zudosierung von Benzol mehrfach in kurzen Zeitabständen redispergiert werden. Überschreitet die Dispergierfrequenz einen bevorzugten Wert von 1/5 s⁻¹, d.h. liegt zwischen zwei (Re-)dispergierungen im Mittel bevorzugt ein Zeitraum (entsprechend einer Verweilzeit) von mehr als 5 Sekunden, so führt die Erhöhung der Raum-Zeit-Ausbeute auf über 5 t / m³ h zu einer Verschlechterung der Produktqualität.

Bei Anwendung der beiden obigen Maßnahmen wurde überraschend gefunden, dass weder die Erhöhung der Starttemperatur alleine (Vergleichsbeispiel 1) noch eine Erhöhung der Dispergierfrequenz alleine (Vergleichsbeispiel 2) es ermöglicht, Nitrobenzol gesichert mit einer Raum-Zeit-Ausbeute von 5,1 t / m³ h herzustellen. In beiden Fällen werden die Grenzwerte wichtiger Prozessparameter wie Abgastemperatur und Salpetersäureumsatz über- bzw. unterschritten. Erst wenn beide Maßnahmen so kombiniert werden, dass das erfindungsgemäße Kriterium erfüllt ist - nämlich mindestens 60% Salpetersäureumsatz in einer Startzone, die höchstens 13 Vol.% des Reaktionsraums einnimmt - kann Nitrobenzol mit einer Raum-Zeit-Ausbeute von mehr als 5 t / m³ h produziert werden (Erfindungsgemäßes Beispiel 1). Wird zusätzlich auch die Zusammensetzung der Mischsäure optimiert, so kann die Raum-Zeit-Ausbeute auch deutlich über 5 t / m³ h angehoben werden (Erfindungsgemäßes Beispiel 2).

Die Erfindung betrifft ein Verfahren zur kontinuierlichen Herstellung von Nitrobenzol durch Nitrierung von Benzol mit Mischsäure in einem Reaktionsraum, bei dem Benzol und die Mischsäure mehrmals ineinander dispergiert werden, dadurch gekennzeichnet, dass

a) die eingesetzte Mischsäure mindestens 3,0 Gew.% Salpetersäure und mindestens 67,0 Gew.% Schwefelsäure, bevorzugt 3,0 bis 5,0 Gew.% Salpetersäure und 67,0 Gew.% bis 75,0 Gew.% Schwefelsäure, jeweils bezogen auf das Gewicht der Mischsäure, enthält, und

b) die Starttemperatur der Reaktion über 100,0°C aber unter 102,0°C, bevorzugt bei mindestens 100,2°C und bei höchstens 101,5°C, liegt, und

c) die nach der ersten Dispergierung erhaltene Emulsion aus Mischsäure und Benzol mindestens 4 mal, bevorzugt 4 bis 8 mal redispergiert wird, bevor der Salpetersäureumsatz 60% beträgt, und

d) innerhalb der ersten 13 Vol.%, bevorzugt innerhalb der ersten 1 bis 13 Vol.%, besonders bevorzugt innerhalb der ersten 5 bis 13 Vol.%, des Reaktionsraums nach der ersten Dispergierung mindestens 60% der eingesetzten Salpetersäure umgesetzt sind.

Durch das erfindungsgemäße Verfahren wird eine Raum-Zeit-Ausbeute von mindestens 5 t Nitrobenzol pro Kubikmeter Volumen des Reaktionsraumes und Stunde erreichbar. In einer bevorzugten Ausführungsform des erfindungsgemäßen Verfahrens wird für die Nitrierung von Benzol in einem Rohrreaktor mit Dispergierelementen eine Raum-

4

Zeit-Ausbeute an Nitrobenzol von über 5 t / m$^3$ h erreicht. Diese bevorzugte Ausführungsform ist gekennzeichnet durch die folgenden zusätzlichen Maßnahmen:

1. die eingesetzte Mischsäure enthält mehr als 3,0 Gew.% Salpetersäure und mehr als 67,0 Gew.% Schwefelsäure, jeweils bezogen auf das Gewicht der Mischsäure, und

2. der Nitriereaktor wird adiabat, also ohne technische Maßnahmen zur Erzeugung von Heiz- oder Kühlenergieströmen an seiner Außenfläche, betrieben.

Eine gute Durchmischung ist für die Reaktion vorteilhaft. Daher wird bevorzugt ein Rohrreaktor eingesetzt, in dem mehrere Dispergierelemente über die Länge des Rohrreaktors verteilt angeordnet sind, die eine intensive Durchmischung und Redispergierung von Benzol, Salpetersäure und Schwefelsäure und Wasser gewährleisten.

Ein solcher Reaktor sowie die Form einsetzbarer Dispergierelemente sind beispielsweise in EP 0 489 211 B1, EP 0 708 076 B1, EP 1 291 078 A2 oder in dem Artikel "Recent advances in the technology of mononitrobenzene manufacture" (Guenkel, A. A.; Maloney, T. W., ACS Symposium Series, 623, Nitration (1996): 223-233) beschrieben. Guenkel und Maloney stellen einen Rohrreaktor vor, dessen neun Dispergierelemente gleichmäßig über die Reaktorlänge verteilt sind und die aus mehreren Halbschalen mit Bohrungen bestehen.

In EP 1 291 078 A2 kommt ein Rohrreaktor mit 3 bis 11 Dispergierelementen aus Tantal zum Einsatz, die jeweils 0,5 bis 4 bar Druckverlust erzeugen und für einen Massenstrom von 1 t/h jeweils 10 bis 25 Öffnungen aufweisen. Bei den Öffnungen kann es sich um Schlitze, Löcher oder Bohrungen handeln. In dem erfindungsgemäßen Verfahren können diese Parameter ebenfalls realisiert werden, um eine Koaleszenz der Phasen zu vermeiden und den Durchmesser der organischen Tröpfchen in der Säurephase klein zu halten. Dabei sind die Dispergierelemente so ausgelegt, dass der mittlere Tröpfchendurchmesser bevorzugt kleiner als 200 μm, besonders bevorzugt kleiner als 120 μm ist.

Eine alternative bevorzugte Ausführungsform des erfindungsgemäßen Verfahrens ist gekennzeichnet durch die folgenden zusätzlichen Maßnahmen:

1. die eingesetzte Mischsäure enthält mehr als 3,0 Gew.% Salpetersäure und mehr als 67,0 Gew.% Schwefelsäure, jeweils bezogen auf das Gewicht der Mischsäure, und

2. die während der Nitrierung anfallenden Reaktionswärme wird teilweise, bevorzugt im Bereich der ersten 13 Vol. %, ganz bevorzugt innerhalb der ersten 20 Vol.%, des Reaktionsraums nach der ersten Dispergierung durch Kühlung abgeführt.

Dadurch wird bewirkt, dass ein Teil der Reaktionswärme abgeführt wird und dadurch die Reaktionsendtemperatur erniedrigt wird. Die Raum-Zeit-Ausbeute kann aufgrund der externen Reaktorkühlung weiter erhöht werden, bis die Reaktionsendtemperatur wieder den technisch zulässigen Grenzwert erreicht.

Die erforderliche Reaktorkühlung kann durch Luftkühlung, durch einen Wärmetauschapparat oder ganz bevorzugt durch eine Mantelkühlung mit Wärmeträger erfolgen. Die Reaktionswärme wird bevorzugt in dem Bereich des Reaktors abgeführt, in dem die Reaktion, d.h. die Wärmeentwicklung zum größten Teile stattfindet. Dieser Bereich ist in dem erfindungsgemäßen Verfahren die Startzone und der unmittelbar daran anschließende Reaktionsraum, d.h., es wird bevorzugt in dem Bereich der ersten 20 Vol.%, ganz bevorzugt innerhalb der ersten 15 Vol.% des Reaktionsraums nach der ersten Dispergierung gekühlt.

Die Anwendung der externen Kühlung erfolgt bevorzugt nur bis zu einem gewissen Maße, denn für die vorliegende Erfindung ist es entscheidend, dass der Salpetersäureumsatz am Ende der Startzone mindestens 60% beträgt. Daher ist es bevorzugt, dass nur in dem Bereich der ersten 20 Vol.%, ganz bevorzugt nur innerhalb der ersten 15 Vol. % des Reaktionsraums nach der ersten Dispergierung gekühlt wird. Dabei kann diese Kühlung sich über den gesamten Bereich der ersten 20 Vol.-% bzw. 15 Vol.-% des Reaktionsraums erstrecken. Sie kann sich aber auch nur auf Teile davon erstrecken, beispielsweise nur auf den Bereich von 0 bis 13 Vol.-% oder auf den Bereich von 2 bis 13 Vol.-% des Reaktionsraums.

In einem Prinzipversuch wurde gezeigt, dass nach einem Herabkühlen der Reaktionsendtemperatur um ein halbes Grad Celsius, die Raum-Zeit-Ausbeute um 0,1 t / m$^3$ h gesteigert werden kann, bis die Reaktionsendtemperatur, die ohne Anwendung der Kühlung vorlag, wieder erreicht wird (Vergleichsbeispiel 5). Dieser Prinzipversuch zeigt, dass unter Anwendung einer externen Kühlung prinzipiell die Raum-Zeit-Ausbeute gesteigert werden kann, ohne dass die Produktqualität negativ beeinflusst wird.

Im erfindungsgemäßen Beispiel 6 wurde diese Beobachtung theoretisch auf einen Versuch übertragen, in dem eine Mischsäure mit mehr als 3,0 Gew.% Salpetersäure und mehr als 67,0 Gew.% Schwefelsäure eingesetzt wurde. Unter Einbeziehung einer effektiven Kühlung können sogar Raum-Zeit-Ausbeuten von 6 t / m$^3$ h erreicht werden.

### Beispiele

#### 1. Vergleichsbeispiel 1 mit erhöhter Starttemperatur:

Benzol wird in einem 7%igen stöchiometrischen Überschuss in eine Mischsäure aus 3,1% Salpetersäure, 67,2% Schwefelsäure und 29,7% Wasser eindosiert. Die Benzolmenge wird so gewählt, dass bezogen auf den Reaktionsraum eine Raum-/Zeit-Ausbeute von 5,1 Tonnen/m$^3$ h erreicht wird. Das erhaltene Gemisch wird bei einer Starttemperatur von 101,4°C in einen Reaktor geführt, in dem die Salpetersäure zu 99,25% abreagiert wird. Innerhalb der ersten 13 Vol.% des Reaktionsraums nach der ersten Dispergierung finden nur 3 Redispergierungen durch Tantaleinbauten statt. Nach 10,9% des Reaktionsraumes wird eine Temperatur von 116,6°C gemessen. Die Temperaturdifferenz von 15,2°C entspricht 49,2% des Temperatursprunges, der bei einer Reaktionsendtemperatur von 132,3°C beobachtet wird.

Temperaturanstieg und Salpetersäureumsatz verlaufen proportional, daher beträgt auch der Umsatz an Salpetersäure nach 10,9 Vol.% des Reaktionsraums 49,2%. Nach Durchlaufen des Reaktors wird die Reaktionsmischung einem Phasentrennapparat zugeführt. Das dort entweichende Abgas weist eine Temperatur von 66,8°C auf. Diese Temperatur entspricht einer Abgasmenge, die aufgrund ihres hohen NOx-Gehaltes unter Sicherheits- und Umweltaspekten nicht über einen längeren Zeitraum tolerierbar ist. Ein dauerhafter Betrieb und weitere Lasterhöhungen sind bei dieser Abgasmenge nicht angeraten.

#### 2. Vergleichsbeispiel 2 mit verbessertem Reaktor mit erhöhter Dispergierung:

Es wird die gleiche Apparatur wie in Beispiel 1 eingesetzt mit dem Unterscheid, dass innerhalb der ersten 13 Vol.% des Reaktionsraums nach der ersten Dispergierung 4 Redispergierungen durch Tantaleinbauten stattfinden. Zum Einbringen des fünften Dispergierelementes wird der Reaktor um 2,5 Vol.% des Reaktorvolumens am Ende der Endzone gekürzt und um 2,5 Vol.% des Reaktorvolumens am Anfang der Startzone verlängert, wodurch das Reaktorvolumen insgesamt konstant bleibt.

Ebenso wie in Vergleichsbeispiel 1 wird Benzol mit Mischsäure zur Reaktion gebracht. Im Unterschied zu Vergleichsbeispiel 1 beträgt die Starttemperatur lediglich 100,0°C. Nach 13,4 Vol.% des Reaktionsraums beträgt die Temperatur der Reaktionsmischung 118,3°C. Die Temperaturdifferenz von 18,3°C entspricht 58,5% des Temperatursprunges, der bei einer Reaktionsendtemperatur von 131,3°C beobachtet wird. Der Umsatz an Salpetersäure nach der Startzone (d.h. hier nach den ersten 13 Vol.% des Reaktionsraums nach der ersten Dispergierung) beträgt 58,5%, der nach dem gesamten Reaktor nur 99,07%. Nach Durchlaufen des Reaktors wird die Reaktionsmischung einem Phasentrennapparat zugeführt. Das am Phasentrennapparat entweichende Abgas weist eine Temperatur von 46,8°C auf. Die aus dem Phasentrennapparat ablaufende Schwefelsäure wird in einem Entspannungsverdampfer aufkonzentriert. Das dort abgetrennte Kondensat enthält noch 0,06 Gew.% Salpetersäure. Dies sind 0,2% der eingesetzten Salpetersäure und stellt einen unvertretbar hohen Verlust an Salpetersäure dar.

#### 3. Erfindungsgemäßes Beispiel 3

Es wird der in Vergleichsbeispiel 2 beschriebene Reaktor eingesetzt.

Ebenso wie in Vergleichsbeispielen 1 und 2 wird Benzol mit Mischsäure zur Reaktion gebracht. Um Nitrobenzol mit einer Raum-Zeit-Ausbeute von über 5 t / m$^3$ h auch gesichert produzieren zu können ohne bei der Qualität Abstriche machen zu müssen, wird nun eine Starttemperatur von 101,4°C angewandt. Nach 13,4 Vol.% des Reaktionsraums beträgt die Temperatur der Reaktionsmischung 120,6°C. Die Temperaturdifferenz von 19,2°C entspricht 62,1% des Temperatursprunges, der bei einer Reaktionsendtemperatur von 132,3°C beobachtet wird. Der Umsatz an Salpetersäure übersteigt in der Startzone (d.h. hier nach den ersten 13 Vol.% des Reaktionsraums nach der ersten Dispergierung) 60%, beträgt nach der Startzone 62,1%, und beträgt nach dem gesamten Reaktor 99,38%. Nach Durchlaufen des Reaktors wird die Reaktionsmischung einem Phasentrennapparat zugeführt. Das am Phasentrennapparat entweichende Abgas weist eine Temperatur von 46,5°C auf. Alle Prozessparameter sind problemlos zu beherrschen.

#### 4. Erfindungsgemäßes Beispiel 4

Die Reaktion wird im gleichen Reaktor wie das erfindungsgemäße Beispiel 3 durchgeführt. Diesmal soll jedoch eine Raum-Zeit-Ausbeute von 5,7 t / m$^3$ h erzielt werden. Dies gelingt, in dem die Startbedingungen so eingestellt werden, dass nach der Startzone ein Salpetersäureumsatz von über 60% eingestellt wird. Dazu wird die Schwefelsäurekonzentration auf 67,0 Gew.% eingestellt und die Starttemperatur auf 100,2°C. Nach 13,4 Vol.% des Reaktionsraums beträgt die Temperatur der Reaktionsmischung 122,0°C. Die Temperaturdifferenz von 21,8°C entspricht 62,1% des Temperatursprunges, der bei einer Reaktionsendtemperatur von 135,3°C beobachtet wird. Der Umsatz an Salpetersäure übersteigt in der Startzone (d.h. hier nach den ersten 13 Vol.% des Reaktionsraums nach der ersten Dispergierung) 60%, beträgt nach der Startzone 62,1% und beträgt nach dem gesamten Reaktor 99,35%. Nach Durchlaufen des Reaktors wird die Reaktionsmischung einem Phasentrennapparat zugeführt. Das am Phasentrennapparat entweichende Abgas weist eine Temperatur von 65,5°C auf. Alle Prozessparameter sind problemlos zu beherrschen.

In den erfindungsgemäßen Beispielen 3 und 4 gelingt es durch die geeignete Kombination der reaktionsbeschleunigenden Faktoren Dispergierung, Schwefelsäurekonzentration und Starttemperatur das erfindungsgemäße Kriterium von mindestens 60% Salpetersäureumsatz innerhalb der ersten 13 Vol-.% des Reaktionsraums nach der ersten Dispergierung von Benzol und Mischsäure zu erfüllen und so Nitrobenzol mit einer Raum-Zeit-Ausbeute von über 5 t / m$^3$ h mit der erforderlichen Qualität herzustellen.

Die Zusammensetzung der Rohnitrobenzolphase im Trennapparat aus den Vergleichsbeispielen 1 und 2 und den erfindungsgemäßen Beispielen 3 und 4, als auch die Temperaturen entlang des Reaktors und die Abgastemperatur, gibt die Tabelle 1 wieder. In allen vier Beispielen wird das gleiche Reaktorvolumen eingesetzt sowie die angegebenen Schwefelsäurekonzentrationen angewandt.

**Tabelle 1**: Versuche zur Realisierung von Raum-Zeit-Ausbeuten von mehr als 5 t / m³ h

| | Vergleichsbeispiel 1 | Vergleichsbeispiel 2 | Erfindungsgemäßes Beispiel 3 | Erfindungsgemäßes Beispiel 4 |
|---|---|---|---|---|
| Raum-Zeit-Ausbeute Nitrobenzol (t / m³ h) | 5,1 | 5,1 | 5,1 | 5,7 |
| Gesamtanzahl der Dispergierungen in der Startzone | 4 | 5 | 5 | 5 |
| Schwefelsäurekonzentration in der Mischsäure (Gew.%) | 67,20 | 67,05 | 67,10 | 67,00 |
| Salpetersäurekonzentration in der Mischsäure (Gew.%) | 3,1 | 3,2 | 3,2 | 3,5 |
| Starttemperatur (°C) | 101,4 | 100,0 | 101,4 | 100,2 |
| Temperatur nach Startzone (°C) | 116,6 | 118,3 | 120,6 | 122,0 |
| Reaktionsendtemperatur(°C) | 132,3 | 131,9 | 132,3 | 135,3 |
| Salpetersäureumsatz nach Startzone (%) | 49,2 | 58,5 | 62,1 | 62,1 |
| Salpetersäureumsatz nach Endzone(%) | 99,25 | 99,07 | 99,38 | 99,35 |
| Abgastemperatur (°C) | 66,8 | 46,8 | 46,5 | 65,5 |
| | | | | |
| Nitrophenole gesamt (ppm) | 2086 | 2100 | 2079 | 2127 |
| Pikrinsäure (ppm) | 288 | 354 | 239 | 306 |
| Dinitrobenzol (ppm) | 153 | 182 | 201 | 224 |
| Bemerkung | Abgastemperatur Nahe Grenzwert | Niedriger Salpetersäure-umsatz | problemlos | problemlos |

**5. Vergleichsbeispiel 5 zum Einfluss der externen Reaktorkühlung:**

Die Reaktion wird im gleichen Reaktor wie die erfindungsgemäßen Beispiele 3 und 4 durchgeführt. Benzol wird in einem 7%igen stöchiometrischen Überschuss in eine Mischsäure aus 2,3 Gew.% Salpetersäure, 68,0 Gew.% Schwefelsäure und 29,7 Gew.% Wasser eindosiert. Die Benzolmenge wird so gewählt, dass bezogen auf das Reaktorvolumen eine Raum/Zeit-Ausbeute von Nitrobenzol von 4,3 t/m$^3$ h erreicht wird. Das erhaltene Gemisch wird bei einer Starttemperatur von 99,8°C in einen Reaktor geführt. Nach 13,4 Vol.% des Reaktionsraumes beträgt die Temperatur der Reaktionsmischung 113,6°C. Die Reaktionsendtemperatur beträgt 123,6°C.

Jetzt wird die externe Reaktorkühlung angewandt, die sich nach der Startzone, jedoch innerhalb der ersten 15 Vol.% des Reaktionsraums nach der ersten Dispergierung befindet. Die angewandte externe Kühlung bewirkt eine Absenkung der Reaktionsendtemperatur um 0,5°C auf 123,1°C. Die Last wird Schrittweise erhöht, bis wieder die Reaktionsendtemperatur erreicht wird, die vor Anwendung der externen Kühlung vorlag. Diese Temperatur wird wieder bei einer Raum-Zeit-Ausbeute von 4,4 t / m$^3$ h erreicht. Wie die Analysenwerte in Tabelle 2 zeigen, wird jetzt bei gleicher Reaktionsendtemperatur und trotz höherer Raum-Zeit-Ausbeute ein Rohnitrobenzol mit gleich bleibender Qualität erhalten.

Auch wenn dieser Prinzipversuch bei Raum-Zeit-Ausbeuten von unter 5 t /m$^3$ h durchgeführt wurde, zeigt er dennoch, dass aufgrund der richtigen Positionierung und Dosierung der externen Kühlung, die Raum-Zeit-Ausbeute gesteigert werden kann, ohne Abstriche bei der Produktqualität oder dem Salpetersäureumsatz machen zu müssen.

**6. Erfindungsgemäßes Beispiel 6: Simulation zum Einfluss der externen Reaktorkühlung:**

Ebenso wie im Vergleichsbeispiel 5 wird die Reaktion mit und ohne externe Kühlung miteinander verglichen. Die diesem Beispiel zugrunde liegenden Daten wurden berechnet.

Führt man in einem Rohrreaktor wie in Beispiel 2 die Nitrierung von Benzol gemäß den Kriterien der vorliegenden Erfindung mit einer Raum-Zeit-Ausbeute von 6,0 t/m$^3$ h aus, so setzt man Benzol in einem 7%igen stöchiometrischen Überschuss in eine Mischsäure aus 3,7 Gew.% Salpetersäure, 67,0 Gew.% Schwefelsäure und 29,3 Gew.% Wasser ein. Für das erhaltene Gemisch wird eine Starttemperatur von 101,5°C angenommen. Aus der Reaktionsenthalpie und der Zusammensetzung des Reaktionsgemisches ergibt sich eine Reaktionsendtemperatur von 137,3 °C. Der Salpetersäureumsatz nach 13 Vol.% des Reaktionsraumes kann zu 67,0% berechnet werden.

Bei einer Reaktionsendtemperatur von 137,3°C ist während der Herstellung von Nitrobenzol mit einer hohen Abgasmenge zu rechnen, die nicht ohne zusätzliche technische Maßnahmen dauerhaft hingenommen werden kann.

Durch die Anwendung einer effizienten externen Reaktorkühlung hingegen kann die Reaktionsendtemperatur zum Beispiel um 3°C gesenkt werden. Wird die Kühlung nach der Startzone, jedoch innerhalb der ersten 15 Vol.% des Reaktionsraums nach der ersten Dispergierung, durchgeführt, so ändert sich der Salpetersäureumsatz in der Startzone nicht. Damit ermöglicht die richtige Positionierung der externen Kühlung die gesicherte Produktion mit hohen Raum-Zeit-Ausbeuten und lässt dennoch hohe Salpetersäureumsätze in der Startzone zu.

Die Zusammensetzung der Rohnitrobenzolphase im Trennapparat aus dem Vergleichsbeispiel 5, als auch die Temperaturen entlang des Reaktors gibt die Tabelle 2 wieder. Dabei steht der Begriff Startzone (wie auch schon im Rahmen der Beispiele 1 bis 4) wieder für die ersten 13 Vol.% des Reaktionsraums nach der ersten Dispergierung.

**Tabelle 2:** Versuche zur externen Reaktorkühlung

|  | Vergleichsbeispiel 5 | | Erfindungsgemäßes Beispiel 6 - Simulation | |
|---|---|---|---|---|
|  | ohne Kühlung | mit Kühlung | ohne Kühlung | mit Kühlung |
| Raum-Zeit-Ausbeute Nitrobenzol (t/ m$^3$ h) | 4,3 | 4,4 | 6,0 | |
| Anzahl der Dispergierungen in der Startzone | 5 | | 5 | |
| Schwefelsäurekonzentration in der Mischsäure (Gew.%) | 68,0 | 68,0 | 67,0 | |
| Salpetersäurekonzentration in der Mischsäure (Gew.%) | 2,27 | 2,30 | 3,71 | |
| Starttemperatur (°C) | 99,8 | | 101,5 | |
| Temperatur nach Startzone (°C) | 113,6 | 114,1 | 125,5 | 125,5 |

(fortgesetzt)

| | Vergleichsbeispiel 5 | | Erfindungsgemäßes Beispiel 6 - Simulation | |
|---|---|---|---|---|
| | ohne Kühlung | mit Kühlung | ohne Kühlung | mit Kühlung |
| Reaktionsendtemperatur (°C) | 123,6 | 123,6 | 137,3 | 134,3 |
| Salpetersäureumsatz nach Startzone (%) | 58,2 | 60,1 | 67,0 | 67,0 |
| Salpetersäureumsatz nach Endzone (%) | 99,44 | 99,45 | 99,6 | 99,6 |
| Nitrophenole gesamt (ppm) | 1915 | 1900 | --- | --- |
| Pikrinsäure (ppm) | 195 | 191 | --- | --- |
| Dinitrobenzol (ppm) | 140 | 138 | --- | --- |

**Patentansprüche**

1. Verfahren zur kontinuierlichen Herstellung von Nitrobenzol durch Nitrierung von Benzol mit Mischsäure in einem Reaktionsraum, bei dem Benzol und die Mischsäure mehrmals ineinander dispergiert werden, **dadurch gekennzeichnet, dass**

   a) die eingesetzte Mischsäure mindestens 3,0 Gew.% Salpetersäure und mindestens 67,0 Gew.% Schwefelsäure, jeweils bezogen auf das Gewicht der Mischsäure, enthält, und
   b) die Starttemperatur der Reaktion über 100,0°C aber unter 102,0°C liegt, und
   c) die nach der ersten Dispergierung erhaltene Emulsion aus Mischsäure und Benzol mindestens 4 mal redispergiert wird, bevor der Salpetersäureumsatz 60% beträgt, und
   d) innerhalb der ersten 13 Vol.% des Reaktionsraums nach der ersten Dispergierung mindestens 60% der eingesetzten Salpetersäure umgesetzt sind.

2. Verfahren nach Anspruch 1, **dadurch gekennzeichnet, dass** die Nitrierung ohne Beheizung oder Kühlung durchgeführt wird.

3. Verfahren nach Anspruch 1, **dadurch gekennzeichnet, dass** die Nitrierung im Bereich der ersten 20 Vol.% des Reaktionsraums gekühlt wird.

4. Verfahren nach Anspruch 1, **dadurch gekennzeichnet, dass** die Nitrierung im Bereich der ersten 15 Vol.% des Reaktionsraums gekühlt wird.

**Claims**

1. Process for the continuous preparation of nitro benzene by nitration of benzene by means of nitrating acid in a reaction space, in which benzene and the nitrating acid are dispersed into one another a plurality of times, **characterised in that**

   a) the nitrating acid used contains at least 3.0% by weight nitric acid and at least 67.0% by weight of sulfuric acid, in each case based on the weight of the nitrating acid, and
   b) the initial temperature of the reaction is above 100.0°C but below 102.0°C and
   c) the emulsion of nitrating acid and benzene obtained after the first dispersion is redispersed at least 4 times before the nitric acid conversion is 60% and
   d) at least 60% of the nitric acid used is reacted within the first 13% by volume of the reaction space after the first dispersion.

2. Process according to Claim 1, **characterized in that** the nitration is carried out without heating or cooling.

3. Process according to Claim 1, **characterized in that** the nitration is cooled in the region of the first 20% by volume of the reaction space.

**4.** Process according to Claim 1, **characterized in that** the nitration is cooled in the region of the first 15% by volume of the reaction space.

**Revendications**

**1.** Procédé pour la production continue de nitrobenzène par nitration avec de l'acide lactique dans un espace de réaction, dans lequel on disperse l'un dans l'autre plusieurs fois le benzène et l'acide lactique, **caractérisé en ce que**

a) l'acide lactique utilisé contient au moins 3,0 % en poids d'acide nitrique et au moins 67,0 % en poids d'acide sulfurique, chacun par rapport au poids de l'acide lactique, et
b) la température initiale de la réaction est supérieure à 100,0 °C, mais inférieure à 102,0 °C, et
c) avant que le degré de conversion de l'acide nitrique n'atteigne 60 % on redisperse au moins 4 fois l'émulsion d'acide lactique et benzène obtenue après la première dispersion, et
d) après la première dispersion au moins 60 % de l'acide nitrique utilisé ont réagi dans les premiers 13 % en volume de l'espace réactionnel.

**2.** Procédé selon la revendication 1, **caractérisé en ce qu'**on effectue la nitration sans chauffage ni refroidissement.

**3.** Procédé selon la revendication 1, **caractérisé en ce qu'**on refroidit la nitration dans la zone des premiers 20 % en volume de l'espace réactionnel.

**4.** Procédé selon la revendication 1, **caractérisé en ce qu'**on refroidit la nitration dans la zone des premiers 15 % en volume de l'espace réactionnel.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- US 2256999 A
- EP 0436443 B1
- EP 0771783 B1
- US 6562247 B2
- EP 0708076 B1
- EP 1816117 A1
- EP 0373966 B1
- EP 0489211 B1
- EP 0779270 B1
- EP 1291078 A2
- US 6242657 B1
- DE 19539205 A1

### In der Beschreibung aufgeführte Nicht-Patentliteratur

- Recent advances in the technology of mononitrobenzene manufacture. **Guenkel, A. A. ; Maloney, T. W.** Nitration. ACS Symposium Series, 1996, vol. 623, 223-233